# EUROPÄISCHE PATENTSCHRIFT

(11) **EP 2 356 442 B1**
(45) Veröffentlichungstag und Bekanntmachung des Hinweises auf die Patenterteilung: **27.03.2013**
(21) Anmeldenummer: 09796995.0
(22) Anmeldetag: 09.12.2009
(51) Int. Cl.: G01N 30/34, G01N 30/46, G01N 33/28, G01N 30/40, G01N 30/38

(54) **VERFAHREN UND ANORDNUNG ZUR GASCHROMATOGRAPHISCHEN ANALYSE EINES GASGEMISCHS**
METHOD AND APPARATUS FOR GAS CHROMATOGRAPHIC ANALYSIS OF A GAS MIXTURE
PROCÉDÉ ET DISPOSITIF POUR L'ANALYSE PAR CHROMATOGRAPHIE EN PHASE GAZEUSE D'UN MÉLANGE GAZEUX

(30) Priorität: 09.12.2008 DE 102008061158
(43) Veröffentlichungstag der Anmeldung: 17.08.2011
(73) Patentinhaber: Siemens Aktiengesellschaft, 80333 München (DE)
(72) Erfinder: GELLERT, Udo, 76756 Bellheim (DE); PROBST, Frank, 76863 Herxheim bei Landau/Pfalz (DE)
(86) Internationale Anmeldenummer: PCT/EP2009/066666
(87) Internationale Veröffentlichungsnummer: WO 2010/066758

(56) Entgegenhaltungen:
- WO-A-02/063290
- JP-A- 6 258 306
- US-A- 3 457 704
- US-A- 3 585 002
- US-A- 4 617 828

## Beschreibung

Die Erfindung betrifft ein Verfahren zur gaschromatographischen Analyse eines Gasgemischs, von dem mittels eines sich in einer ersten Ventilstellung befindenden Dosierventils eine Probe in ein Dosiervolumen und in einer zweiten Ventilstellung die Probe mittels eines Trägergases aus dem Dosiervolumen durch eine Trenneinrichtung geleitet wird, an deren Ende mindestens eine dort ankommende und aus der Probe getrennte interessierende Gaskomponente mittels eines Detektors erfasst und anhand des von dem Detektor gelieferten Detektorsignals quantitativ bestimmt wird, wobei, nachdem die mindestens eine interessierende Gaskomponente zumindest einen Teil der Trenneinrichtung durchlaufen hat, dieser Teil der Trenneinrichtung und das Dosierventil mit einem von dem einen Trägergas verschiedenen weiteren Trägergas gespült werden und anschließend eine weitere Probe aus dem Dosiervolumen in die Trenneinrichtung geleitet wird.

Die Erfindung betrifft ferner eine Analyse vorrichtung zur Durchführung des Verfahrens.

Ein derartiges Verfahren und eine zugehörige Analyse vorrichtung sind aus der US 34577704 A bekannt. Dort wird, nachdem die interessierenden Gaskomponenten einen Teil der Trenneinrichtung durchlaufen haben, dem Trägergas ein zusätzliches Gas progressiv beigemischt, so dass die Zusammensetzung der mobilen Phase und die Retention der Gaskomponenten der Probe in der Trenneinrichtung geändert werden.

Manche Gaskomponenten, z. B. Wasserstoff, sind mit gängigen Trägergasen, z. B. Helium, nicht eindeutig bestimmbar. Wird stattdessen ein anderes Trägergas verwendet, können solche Gaskomponenten zwar wieder eindeutig bestimmt werden, jedoch sind dann häufig andere Gaskomponenten des zu analysierenden Gasgemischs nicht mehr oder nur mit verringerter Genauigkeit bzw. Empfindlichkeit bestimmbar.

Aus der JP 6-258306 A ist es bekannt, zur Erhöhung der Messempfindlichkeit bei der gaschromatographischen Analyse eines Wasserstoff und Kohlenwasserstoffe enthaltenden Gasgemischs das verwendete Trägergas während der Analyse zu wechseln. Dabei wird zunächst die Probe mittels Stickstoff als erstem Trägergas durch eine aus zwei Trennabschnitten bestehende Trenneinrichtung geleitet, an deren Ende der Wasserstoff detektiert und quantitativ bestimmt wird. Anschließend werden die Kohlenwasserstoffe, nachdem sie in den zweiten Trennabschnitt gelangt sind, durch diesen mittels Helium als zweitem Trägergas zu dem Detektor geleitet, während der erste Trennabschnitt mit dem Stickstoff rückgespült wird. Die Trägergasumschaltung inmitten der Trenneinrichtung kann problematisch sein, weil die Kohlenwasserstoffe zunächst noch in dem Stickstoff enthalten sind und erst im weiteren Verlauf des zweiten Trennabschnitts von dem Helium erfasst werden.

Aus der CN 1885031 A ist ein Verfahren zur gaschromatographischen Analyse eines Sauerstoff, Stickstoff und Wasserstoff enthaltenden Gasgemischs bekannt, bei dem zur Bestimmung des Wasserstoffs Stickstoff oder Argon als erstes Trägergas und zur Bestimmung der anderen Gaskomponenten Wasserstoff als zweites Trägergas verwendet wird. Dazu wird das Gasgemisch mittels eines sich in einer ersten Ventilstellung befindenden Dosierventils durch zwei verschiedene Dosiervolumina geführt, die somit zwei Proben des Gasgemischs bereitstellen. Gleichzeitig werden eine erste Trenneinrichtung mit dem ersten Trägergas und eine zweite Trenneinrichtung mit dem zweiten Trägergas gespült. In der zweiten Ventilstellung wird die erste Probe von dem ersten Trägergas durch die erste Trenneinrichtung und die zweite Probe von dem zweiten Trägergas durch die zweite Trenneinrichtung geleitet. Am Ende beider Trenneinrichtungen ist eine Umschalteinrichtung vorgesehen, über die entweder die aus der ersten Trenneinrichtung austretenden getrennten Gaskomponenten oder die aus der zweiten Trenneinrichtung austretenden getrennten Gaskomponenten einem Detektor zugeführt werden. Durch die Umschalteinrichtung am Ende der Trenneinrichtungen können die getrennten Gaskomponenten gestört werden, was die Detektionsgenauigkeit vermindert. Außerdem muss sichergestellt werden, dass die Gaskomponenten aus den verschiedenen Trenneinrichtungen in genügend großem zeitlichem Abstand an der Umschalteinrichtung ankommen. Schließlich ist der apparative Aufwand erheblich, da tatsächlich zwei Gaschromatographen verwendet werden, die zwar ein gemeinsames Dosierventil und einen gemeinsamen Detektor haben, wobei aber der Aufbau des Dosierventils mit den zwei Dosiervolumina aufwendiger als bei einem einzelnen Gaschromatographen ist und die zusätzliche Umschalteinrichtung benötigt wird. Es bietet sich daher auch die alternative Möglichkeit an, ein und dasselbe Gasgemisch in zwei verschiedenen Gaschromatographen mit zwei verschiedenen Trägergasen zu analysieren und die Messergebnisse beider Analysen zusammenzuführen (US 3585002 A).

Der Erfindung liegt daher die Aufgabe zugrunde, eine genaue gaschromatographische Analyse mit minimalem technischem Aufwand zu ermöglichen.

Gemäß der Erfindung wird die Aufgabe dadurch gelöst, dass bei dem Verfahren der eingangs angegebenen Art die weitere Probe mittels des weiteren Trägergases aus dem Dosiervolumen in die Trenneinrichtung geleitet wird.

Zunächst wird also eine Probe des Gasgemischs mit dem einen (ersten) Trägergas durch die Trenneinrichtung geleitet. Spätestens dann, wenn die interessierende Gaskomponente am Ende der Trenneinrichtung detektiert worden ist, wird die Trenneinrichtung mit dem zweiten Trenngas gespült und anschließend mittels des weiteren (zweiten) Trägergases eine weitere Probe des Gasgemischs aus dem Dosiervolumen in die Trenneinrichtung geleitet. Diese weitere Probe, die z. B. während der mit dem ersten Trenngas erfolgenden Analyse in das Dosiervolumen eingeleitet werden kann, kann ebenfalls am Ende der Trenneinrichtung detektiert werden. Es ist aber auch möglich, interessierende Gaskomponenten der weiteren Probe vorher mittels eines zwischen dem Dosierventil und dem Detektor im Verlauf der Trenneinrichtung liegenden weiteren Detektors zu erfassen oder mittels einer im Verlauf der Trenneinrichtung liegenden Gasumschalteinrichtung, wie sie z. B. aus der WO 03/083467 A2 bekannt ist, aus der Trenneinrichtung auszuschleusen und einer weiteren Trenneinrichtung mit nachgeordnetem weiteren Detektor zuzuführen. Eine solche Gasumschalteinrichtung ermöglicht es auch, den zwischen dem Dosierventil und der Gasumschalteinrichtung liegenden Teil der Trenneinrichtung bereits dann mit dem zweiten Trägergas zu spülen, nachdem die von dem ersten Trägergas transportierte interessierende Gaskomponente die Gasumschalteinrichtung passiert hat. Das erste Trägergas wird dann an der Gasumschalteinrichtung in den hinteren Teil der Trenneinrichtung eingeleitet, um darin die interessierende Gaskomponente weiter zu transportieren.

Bei einem Wasserstoff und Kohlenwasserstoffe enthaltenden Gasgemisch wird vorzugsweise zur Trennung und Detektion des Wasserstoffs Argon oder Stickstoff und zur Trennung und Detektion der Kohlenwasserstoffe Helium als Trägergas verwendet.

Zur Realisierung der Trägergasumschaltung sind eine erste und zweite Trägergasquelle sowie eine steuerbare Ventilschaltung vorgesehen, die je nach Schaltstellung entweder die erste oder die zweite Trägergasquelle mit dem Gaschromatographen verbindet. Die Ventilschaltung enthält vorzugsweise drei steuerbare Dreiwegventile, von denen ein erstes Ventil und ein zweites als Dreiwegeventil ausgebildetes Ventil zwischen der ersten Trägergasquelle und dem Gaschromatographen und ein drittes Ventil und das zweite Ventil zwischen der zweiten Trägergasquelle und dem Gaschromatographen liegen. Dadurch wird sichergestellt, dass das jeweils nicht verwendete Trägergas immer durch zwei Ventile gesperrt ist und die beiden Trägergase immer durch zwei Ventile und den dazwischen liegenden Gasweg getrennt sind. Vorzugsweise sind das erste und dritte Ventil als Dreiwegeventile ausgebildet, wobei sie in der das jeweilige Trägergas sperrenden Ventilstellung den Gasweg zwischen ihnen und dem zweiten Dreiwegeventil über jeweils einen Auslass entlüften. Damit wird der Druck zwischen den beiden Sperrstellen für die beiden Trägergase auf den Umgebungsdruck begrenzt, so dass sich die beiden Trägergase auch im einfachen Fehlerfall nicht vermischen können.

Zur weiteren Erläuterung der Erfindung wird im Folgenden auf die Figuren der Zeichnung Bezug genommen; Im Einzelnen zeigen:
- Figur 1: ein einfaches Beispiel eines Gaschromatographen, der über eine Ventilschaltung nacheinander mit zwei unterschiedlichen Trägergasen versorgt wird und
- Figur 2: ein weiteres Beispiel eines mit zwei Trägergasen versorgten Gaschromatographen.

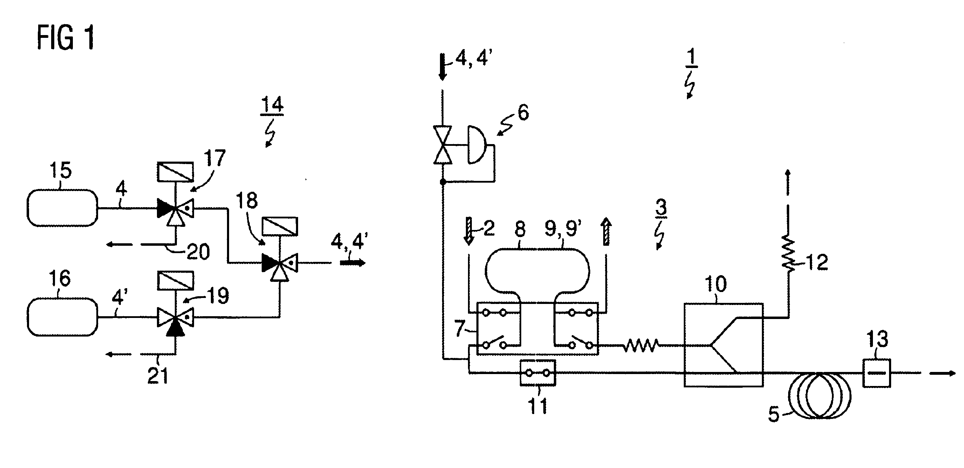

Bei dem in Figur 1 gezeigten Gaschromatograph 1 wird ein zu analysierendes Gasgemisch 2 nach Entnahme aus einem technischen Prozess einer Dosiereinrichtung 3 zugeführt. Die Dosiereinrichtung 3 dient dazu, zu einem vorgegebenen Zeitpunkt eine vorgegebene Dosiermenge des Gasgemischs 2 in Form eines kurzen und scharf begrenzten Probenpfropfes in einen Trägergasstrom 4 einzuschleusen und einer Trenneinrichtung 5 zuzuführen. Der Druck und damit die Strömungsgeschwindigkeit des Trägergases 4 werden mittels eines Druckreglers 6 geregelt. Die Dosiereinrichtung 3 weist ein Dosierventil 7 auf, welches in einer hier gezeigten ersten Schaltstellung das Gasgemisch 2 in ein Dosiervolumen 8 leitet. In einer zweiten Schaltstellung wird das Dosiervolumen 8 in den Weg für das Trägergas 4 geschaltet, das die in dem Dosiervolumen 8 enthaltene Probe 9 des Gasgemischs 2 einem Injektor 10 zuführt. Solange ein Magnetventil 11 geöffnet ist, fließt das Trägergas 4 durch das Magnetventil 11 und den Injektor 10 in die Trenneinrichtung 5, während die Probe 9 aus dem Dosiervolumen 8 über eine Strömungsdrossel 12 nach außen abgeleitet wird. Wird das Magnetventil 11 für eine vorgegebene Dauer geschlossen, so wird in dem Injektor 10 aus der Probe 9 ein Teil abgezweigt und als scharf begrenzter Probenpfropf in den Trägergasstrom 4 zur Trenneinrichtung 5 eingeschleust. Die in dem Probenpfropf enthaltenen Gaskomponenten der Gasgemischs 2 werden beim Durchströmen der Trenneinrichtung 5 getrennt und erscheinen nacheinander am Ende der Trenneinrichtung 5, wo sie mittels eines Detektors 13 detektiert und durch Auswertung des von dem Detektor 13 gelieferten Detektorsignals quantitativ bestimmt werden. Bei dem hier gezeigten einfachsten Fall besteht die Trenneinrichtung 5 aus einer Trennsäule.

Der Druckregler 6 des Gaschromatographen 1 ist eingangsseitig über eine steuerbare Ventilschaltung 14 an zwei Trägergasquellen 15 und 16 angeschlossen, von denen die eine das Trägergas 4 und die andere ein davon verschiedenes weiteres Trägergas 4' bereitstellt. Je nach Schaltstellung der Ventilanordnung 14 wird entweder das eine Trägergas 4 oder das weitere Trägergas 4' in den Gaschromatograph 1 eingeleitet. Dazu weist die Ventilanordnung 14 ein erstes Dreiwegeventile 17 und ein zweites Dreiwegeventil 18 auf, die hintereinander zwischen der ersten Trägergasquelle 15 und dem Gaschromatoraphen 1 liegen, und ein drittes Dreiwegeventil 19 zwischen der zweiten Trägergasquelle 16 und dem zweiten Dreiwegeventil 18. Das erste Dreiwegeventil 17 und das dritte Dreiwegeventil 19 weisen jeweils einen Auslass 20 bzw. 21 auf, über den der Gasweg zwischen ihnen und dem zweiten Dreiwegeventil 18 entlüftet wird, wenn sie sich in der das jeweilige Trägergas 4 bzw. 4' sperrenden Ventilstellung befinden. Bei der gezeigten Ventilstellung strömt das erste Trägergas 4 aus der ersten Trägergasquelle 15 durch die Ventile 17 und 18 in den Gaschromatographen 1, während das Ventil 19 das zweite Trägergas 4' sperrt und den Gasweg zwischen den Ventilen 18 und 19 über den Auslass 21 entlüftet. In der anderen, hier nicht gezeigten Ventilstellung strömt das zweite Trägergas 4' aus der zweiten Trägergasquelle 16 durch die Ventile 19 und 18 in den Gaschromatographen 1, während das Ventil 17 das erste Trägergas 4 sperrt und den Gasweg zwischen den Ventilen 18 und 17 über den Auslass 20 entlüftet. Die Trägergase 4 und 4' sind daher immer durch zwei Ventile und den dazwischen liegenden Gasweg getrennt, womit der Druck zwischen den beiden Sperrstellen auf den Umgebungsdruck begrenzt wird. Damit wird sichergestellt, dass sich die beiden Trägergase 4 und 4' auch im einfachen Fehlerfall nicht vermischen können. Um zu verhindern, dass Luft in den Gasweg zwischen den beiden Sperrstellen eindringen kann, sind die Auslässe 20, 21 in Form von ausreichend langen Gasleitungen mit einem entsprechend langen Diffusionsweg für die Luft ausgebildet.

In einem ersten Schritt werden die Gaswege des Chromatographen 1 mit dem Trägergas 4 gespült bzw. befüllt. Anschließend wird das Dosiervolumen 8 mit einer ersten Probe 9 des Gasgemischs 2 gefüllt und die Probe 9 in den Trägergasstrom 4 injiziert. Von den beim Durchströmen der Trenneinrichtung 5 getrennten Komponenten der ersten Probe 9 werden alle oder einzelne ausgewählte Probenkomponenten am Ende der Trenneinrichtung 5 detektiert und quantitativ bestimmt. Danach werden die Gaswege des Chromatographen 1 mit dem zweiten Trägergas 4 gespült bzw. befüllt. Anschließend wird das Dosiervolumen 8 mit einer zweiten Probe 9' des Gasgemischs 2 gefüllt und die zweite Probe 9' in den Trägergasstrom 4' injiziert. Von den beim Durchströmen der Trenneinrichtung 5 getrennten Komponenten der zweiten Probe 9' werden alle oder einzelne ausgewählte Probenkomponenten am Ende der Trenneinrichtung 5 detektiert und quantitativ bestimmt.

Wird in der gezeigten Schaltstellung der Ventilanordnung 14 nur das Ventil 18 umgeschaltet, baut sich der Trägergasdruck aus dem Gaschromatographen 1 rückwärts über den Auslass 21 des Ventils 19 ab. Wird dieser Vorgang mehrmals hintereinander wiederholt, dann können mit dem aktuell aufgeschalteten Trägergas 4 auch indirekt bespülte Stellen im Gasweg von dem vorher aufgeschalteten Trägergas 4' gereinigt werden. Auf diese Weise lässt sich die nach jeder Trägergasumschaltung benötigte Spülzeit verkürzen.

Besteht das Gasgemisch 2 aus Wasserstoff und Kohlenwasserstoffen, werden z. B. Argon oder Stickstoff als erstes Trägergas 4 (oder zweites Trägergas 4') zur Trennung und Detektion des Wasserstoffs und Helium als zweites Trägergas 4' (oder erstes Trägergas 4) zur Trennung und Detektion der Kohlenwasserstoffe verwendet.

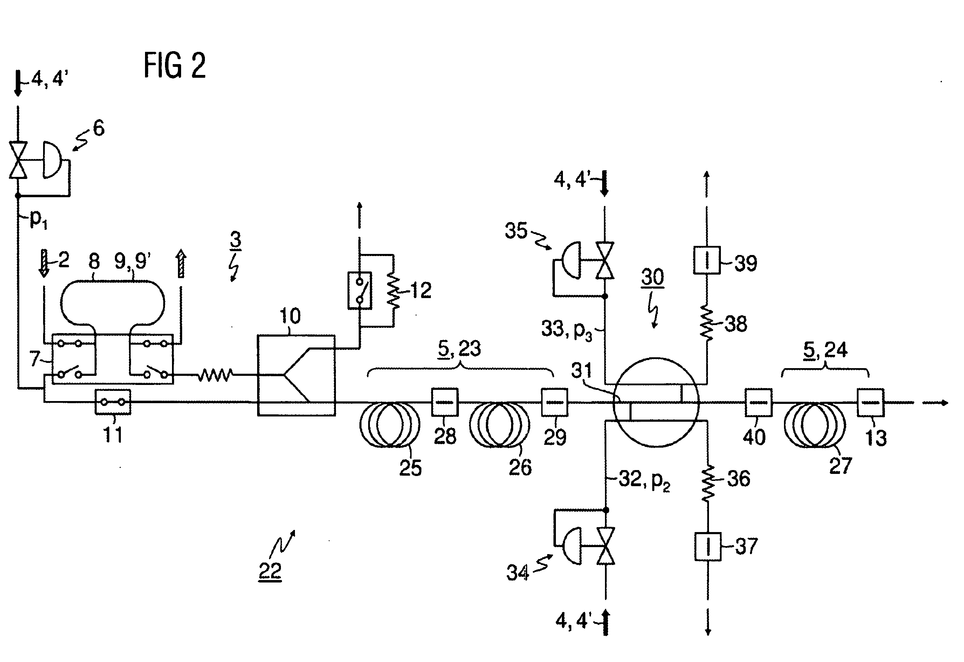

Bei dem in Figur 2 gezeigten Beispiel eines Gaschromatographen 22 besteht die Trenneinrichtung 5 aus zwei aufeinander folgenden Trennanordnungen 23 und 24 in Form von hintereinander geschalteten Trennsäulen 25, 26 und 27, die unterschiedliche Trenneigenschaften aufweisen und unterschiedlich temperierbar sind. Am Ende jeder Trennsäule 25, 26, 27 ist jeweils ein Detektor 28, 29 bzw. 13 zur Detektion von vorgegebenen und bis dahin vollständig getrennten Komponenten des Gasgemischs 2 angeordnet. Zwischen der ersten Trennanordnung 23 und der zweiten Trennanordnung 24 ist eine Gasumschalteinrichtung 30 eingefügt. Diese weist einen die beiden Trennanordnungen 23 und 24 miteinander verbindenden Hauptgasweg 31 und zwei Hilfsgaswege 32 und 33 auf, von denen der Hilfsgasweg 32 an einer näher zur Trennanordnung 23 liegenden Stelle und der Hilfsgasweg 33 an einer näher zur Trennanordnung 24 liegenden Stelle mit dem Hauptgasweg 31 verbunden ist. Beide Hilfsgaswege 32 und 33 werden einlassseitig jeweils über einen Druckregler 34 bzw. 35 mit einem der Trägergase 4, 4' beströmt und weisen auslassseitig jeweils einen Strömungswiderstand 36 bzw. 37 sowie einen Detektor 38 bzw. 39 auf. Wenn der von dem Druckregler 6 bereitgestellte Trägergasdruck p₁ größer als der Trägergasdruck p₂ im Hilfsgasweg 32 und der Druck p₂ wiederum größer als der Trägergasdruck p₃ im Hilfsgasweg 33 eingestellt ist, wird das zunächst ausgewählte erste Trägergas 4 mit der Probe 9 nacheinander durch die Trennsäulen 25, 26 und 27 geleitet, wobei getrennte Probenkomponenten hinter jeder Trennsäulen 25, 26, 27 detektiert werden können. Sobald die letzte noch zu detektierende Probenkomponente die Gasumschalteinrichtung 30 passiert hat, was durch einen zusätzlichen Detektor 40 am Ausgang des Hauptgasweges 31 detektierbar ist, können die Dosiereinrichtung 3 und die erste Trennanordnung 23 bereits mit dem zweiten Trägergas 4' gespült werden, während die Probe 9 von dem ersten Trägergas 4 durch die zweite Trennanordnung 24 geleitet wird. Dazu wird der Trägergasdruck p₃ im Hilfsgasweg 33 größer als der Trägergasdruck p₂ im Hilfsgasweg 32 eingestellt, so dass das ein größerer Teil des Trägergases 4 aus dem Hilfsgasweg 33 in den Hauptgasweg 31 gelangt und sich dort wiederum in einen die Probe 9 durch die zweite Trennanordnung 24 leitenden Teilstrom und einen rückwärts in den Hilfsgasweg 32 fließenden Teilstrom verzweigt. Die erste Trennanordnung 23 ist dann fluidisch von der zweiten Trennanordnung 24 entkoppelt und kann über den Druckregler 6 mit dem zweiten Trenngas 4' gespült werden. Wenn die Probe 9 in dem ersten Trägergas 4 am Ende der zweiten Trennanordnung 24 detektiert worden ist, kann auch die zweite Trennanordnung 24 mit dem zweiten Trenngas 4' gespült werden. Anschließend kann die zweite Probe 9' in den zweiten Trägergasstrom 4' injiziert und im weiteren Verlauf der Trenneinrichtung 5 getrennt und detektiert werden.

## Patentansprüche

1. Verfahren zur gaschromatographischen Analyse eines Gasgemischs, von dem mittels eines sich in einer ersten Ventilstellung befindenden Dosierventils (7) eine Probe (9) in ein Dosiervolumen (8) und in einer zweiten Ventilstellung die Probe (9) mittels eines Trägergases (4) aus dem Dosiervolumen (8) durch eine Trenneinrichtung (5) geleitet wird, an deren Ende mindestens eine dort ankommende und aus der Probe (9) getrennte interessierende Gaskomponente mittels eines Detektors (13) erfasst und anhand des von dem Detektor (13) gelieferten Detektorsignals quantitativ bestimmt wird, wobei, nachdem die mindestens eine interessierende Gaskomponente zumindest einen Teil (18) der Trenneinrichtung (5) durchlaufen hat, dieser Teil (18) der Trenneinrichtung (5) und das Dosierventil (7) mit einem von dem einen Trägergas (4) verschiedenen weiteren Trägergas (4') gespült werden und anschließend eine weitere Probe (9') aus dem Dosiervolumen (8) in die Trenneinrichtung (5) geleitet wird, **dadurch gekennzeichnet, dass** die weitere Probe (9') mittels des weiteren Trägergases (4') aus dem Dosiervolumen (8) in die Trenneinrichtung (5) geleitet wird.

2. Verfahren nach Anspruch 1, **dadurch gekennzeichnet, dass** mittels des Detektors (13) am Ende der Trenneinrichtung (5) mindestens eine dort ankommende und aus der weiteren Probe (9') getrennte interessierende weitere Gaskomponente erfasst und anhand des von dem Detektor (13) gelieferten Detektorsignals quantitativ bestimmt wird.

3. Verfahren nach Anspruch 1 oder 2, **dadurch gekennzeichnet, dass** die weitere Gaskomponente mittels eines zwischen dem Dosierventil (7) und dem Detektor (13) im Verlauf der Trenneinrichtung (5) liegenden weiteren Detektors erfasst wird.

4. Verfahren nach Anspruch 1 oder 2, **dadurch gekennzeichnet, dass** die weitere Gaskomponente mittels einer zwischen dem Dosierventil (7) und dem Detektor (13) im Verlauf der Trenneinrichtung (5) liegenden Gasumschalteinrichtung (30) aus der Trenneinrichtung (5) ausgeschleust und einer weiteren Trenneinrichtung mit nachgeordnetem weiteren Detektor zugeführt wird.

5. Verfahren nach einem der vorangehenden Ansprüche, **dadurch gekennzeichnet, dass**, nachdem die mindestens eine interessierende Gaskomponente eine zwischen dem Dosierventil (7) und dem Detektor (13) im Verlauf der Trenneinrichtung (5) liegende Gasumschalteinrichtung (30) passiert hat, der vor der Gasumschalteinrichtung liegende Teil der Trenneinrichtung (5) mit dem weiteren Trägergas (4') gespült wird und dass das eine Trägergas (4) zur Weiterleitung der mindestens einen interessierenden Gaskomponente über die Gasumschalteinrichtung in den hinter der Gasumschalteinrichtung liegenden Teil der Trenneinrichtung (5) eingespeist wird.

6. Verfahren nach einem der vorangehenden Ansprüche, **dadurch gekennzeichnet, dass** bei einem Wasserstoff und Kohlenwasserstoffe enthaltenden Gasgemisch zur Trennung und Detektion des Wasserstoffs Argon oder Stickstoff und zur Trennung und Detektion der Kohlenwasserstoffe Helium als Trägergas (4, 4') verwendet wird.

7. Analyse vorrichtung zur Durchführung des Verfahrens nach einem der vorangehenden Ansprüche mit einer ersten Trägergasquelle (15), einer zweiten Trägergasquelle (16) und einer steuerbaren Ventilschaltung (14), die je nach Schaltstellung entweder die erste Trägergasquelle (15) oder die zweite Trägergasquelle (16) mit einem Gaschromatographen (1) verbindet.

8. Analyse vorrichtung nach Anspruch 7, **dadurch gekennzeichnet, dass** die Ventilschaltung (14) drei steuerbare Dreiwegventile (17, 18, 19) enthält, von denen ein erstes Ventil (17) und ein als Dreiwegeventil ausgebildetes zweites Ventil (18) zwischen der ersten Trägergasquelle (15) und dem Gaschromatographen (1) und ein drittes Ventil (19) und das zweite Ventil (18) zwischen der zweiten Trägergasquelle (16) und dem Gaschromatographen (1) liegen.

9. Analyse vorrichtung nach Anspruch 8, **dadurch gekennzeichnet, dass** das erste Ventil (17) und das dritte Ventil (19) als Dreiwegeventile ausgebildet sind und in der das jeweilige Trägergas (4, 4') sperrenden Ventilstellung den Gasweg zwischen ihnen und dem zweiten Dreiwegeventil (18) über jeweils einen Auslass (20, 21) entlüften.

## Claims

1. Method for the gas chromatographic analysis of a gas mixture, a sample (9) of which is sent to a dosing volume (8) by means of a dosing valve (7) located in a first valve position and, in a second valve position, the sample (9) is sent by means of a carrier gas (4) from the dosing volume (8) through a separating device (5), at the end of which at least one gas component of interest arriving there and separated from the sample (9) is detected by means of a detector (13) and is quantitatively determined on the basis of the detector signal supplied by the detector (13), wherein, once the at least one gas component of interest has passed through at least part (18) of the separating device (5), this part (18) of the separating device (5) and the dosing valve (7) are flushed with a further carrier gas (4'), different from the one carrier gas (4), and then a further sample (9') is sent from the dosing volume (8) into the separating device (5), **characterized in that** the further sample (9') is sent from the dosing volume (8) into the separating device (5) by means of the further carrier gas (4').

2. Method according to Claim 1, **characterized in that**, by means of the detector (13) at the end of the separating device (5), at least one further gas component of interest arriving there and separated from the further sample (9') is detected and quantitatively determined on the basis of the detector signal supplied by the detector (13).

3. Method according to Claim 1 or 2, **characterized in that** the further gas component is detected by means of a further detector lying between the dosing valve (7) and the detector (13) in the path of the separating device (5).

4. Method according to Claim 1 or 2, **characterized in that** the further gas component is discharged by means of a gas changeover device (30), lying between the dosing valve (7) and the detector (13) in the path of the separating device (5),from the separating device (5) and fed to a further separating device with a downstream further detector.

5. Method according to one of the preceding claims, **characterized in that**, once the at least one gas component of interest has passed a gas changeover device (30) lying between the dosing valve (7) and the detector (13) in the path of the separating device (5), the part of the separating device (5) lying upstream of the gas changeover device is flushed with the further carrier gas (4') and **in that** the one carrier gas (4) is fed by way of the gas changeover device into the part of the separating device (5) lying downstream of the gas changeover device to pass on the at least one gas component of interest.

6. Method according to one of the preceding claims, **characterized in that**, in the case of a gas mixture containing hydrogen and hydrocarbons, argon or nitrogen is used as the carrier gas (4) for separating and detecting the hydrogen and helium is used as the carrier gas (4') for separating and detecting the hydrocarbons.

7. Analysis device for carrying out the method according to one of the preceding claims, with a first carrier gas source (15), a second carrier gas source (16) and a controllable valve switching means (14), which, depending on the switching position, connects either the first carrier gas source (15) or the second carrier gas source (16) to a gas chromatograph (1).

8. Analysis device according to Claim 7, **characterized in that** the valve switching means (14) includes three controllable three-way valves (17, 18, 19), of which a first valve (17) and a second valve (18) lie between the first carrier gas source (15) and the gas chromatograph (1) and a third valve (19) and the second valve (18) lie between the second carrier gas source (16) and the gas chromatograph (1).

9. Analysis device according to Claim 8, **characterized in that** the first valve (17) and the third valve (19) are formed as three-way valves and, in the valve position shutting off the respective carrier gas (4, 4'), vent the gas path between them and the second three-way valve (18) by way of an outlet for each (20, 21).

## Revendications

1. Procédé d'analyse par chromatographie en phase gazeuse d'un mélange gazeux, dont on envoie au moyen d'une vanne (16) doseuse se trouvant dans une première position, un échantillon (9) dans un volume (8) de dosage et, dans une deuxième position de la vanne d'échantillon (9) au moyen d'un gaz (4) porteur du volume (8) de dosage, dans un dispositif (5) de séparation à l'une des extrémités duquel au moins un constituant gazeux intéressant y arrivant et séparé de l'échantillon (9) est détecté au moyen d'un détecteur (13) et est déterminé quantitativement au moyen du signal de détecteur fourni par le détecteur (13), dans lequel, après que le au moins un constituant gazeux intéressant a traversé au moins une partie (18) du dispositif (5) de séparation, cette partie (18) du dispositif (5) de séparation et la vanne (7) de dosage est balayée par un autre gaz (4') porteur différent du gaz (4) porteur et ensuite un autre échantillon (9') est envoyé à partir du volume (8) de dosage pour le dispositif (5) de séparation, **caractérisé en ce que** l'autre échantillon (9') est envoyé du volume (8) de dosage dans le dispositif (5) de séparation au moyen de l'autre gaz (4') porteur.

2. Procédé suivant la revendication 1, **caractérisé en ce qu'**au moyen du détecteur (13) à la fin du dispositif (5) de séparation on détecte au moins un autre constituant gazeux intéressant y arrivant et séparé de l'autre échantillon (9') et on le détermine quantitativement au moyen du signal du détecteur fourni par le détecteur (13).

3. Procédé suivant la revendication 1 ou 2, **caractérisé en ce que** l'on détecte l'autre constituant gazeux au moyen d'un autre détecteur se trouvant dans le trajet du dispositif (5) de séparation entre la vanne (7) de dosage et le détecteur (13).

4. Procédé suivant la revendication 1 ou 2, **caractérisé en ce que** l'on fait sortir du dispositif (5) de séparation l'autre constituant gazeux, au moyen d'un dispositif (30) de commutation de gaz se trouvant dans le trajet du dispositif (5) de séparation entre la vanne (7) de dosage et le détecteur (13).

5. Procédé suivant l'une des revendications précédentes, **caractérisé en ce qu'**après que le au moins un constituant gazeux intéressant a passé dans un dispositif (30) de commutation de gaz se trouvant dans le trajet du dispositif (5) de séparation entre la vanne (7) de dosage et le détecteur (13), on balaye par un autre gaz (4') porteur la partie du dispositif (5) de séparation se trouvant avant le dispositif de commutation de gaz et **en ce que** l'on injecte le un gaz (4) porteur pour l'acheminement du au moins un constituant gazeux intéressant par le dispositif de commutation de gaz, dans la partie du dispositif (5) de séparation se trouvant derrière le dispositif de commutation de gaz.

6. Procédé suivant l'une des revendications précédentes, **caractérisé en ce que**, pour un mélange gazeux contenant de l'hydrogène et des hydrocarbures, on utilise pour la séparation et la détection de l'hydrogène, de l'argon ou de l'azote et pour la séparation et la détection des hydrocarbures de l'hélium comme gaz (4, 4') porteur.

7. Dispositif d'analyse pour effectuer le procédé suivant l'une des revendications précédentes, comprenant une première source (15) de gaz porteur, une deuxième source (16) de gaz porteur et un circuit (14) de vanne pouvant être commandée qui, suivant la position de commutation, met la première source de gaz porteur ou la deuxième source (16) de gaz porteur en communication avec le chromatographe (1) en phase gazeuse.

8. Dispositif d'analyse suivant la revendication 7, **caractérisé en ce que** le circuit (14) de vanne comporte trois vannes (17, 18, 19) à trois voies, dont une première vanne (17) et une deuxième vanne (18) constituées en vanne à trois voies se trouvent entre la première source (15) de gaz porteur et le chromatographe (1) en phase gazeuse et une troisième vanne (19) et la deuxième vanne (18) se trouvent entre la deuxième source (16) de gaz porteur et le chromatographe (1) en phase gazeuse.

9. Dispositif d'analyse suivant la revendication 8, **caractérisé en ce que** la première vanne (17) et la troisième vanne (19) sont constituées en vannes à trois voies et dans lequel la position de vanne bloquant le gaz (4') porteur respectif met à l'atmosphère le trajet de gaz entre elles et la deuxième vanne (18) à trois voies par respectivement une sortie (20, 21).
